(19) Europäisches Patentamt
European Patent Office
Office européen des brevets

(11) **EP 1 582 199 B1**

(12) **FASCICULE DE BREVET EUROPEEN**

(45) Date de publication et mention
de la délivrance du brevet:
**26.09.2012 Bulletin 2012/39**

(51) Int Cl.:
*A61K 8/26* *(2006.01)*    *A61Q 5/04* *(2006.01)*

(21) Numéro de dépôt: **05300229.1**

(22) Date de dépôt: **30.03.2005**

(54) **Composition de deformation permanente des cheveux comprenant au moins un materiau modifie par incorporation d'un metal**

Mindestens ein mit Metal modifiziertes Material enthaltende Zusammensetzung zur dauerhaften Verformung von Haaren.

Hair-perming composition comprising at least one metal modified material

(84) Etats contractants désignés:
**AT BE BG CH CY CZ DE DK EE ES FI FR GB GR HU IE IS IT LI LT LU MC NL PL PT RO SE SI SK TR**

(30) Priorité: **31.03.2004 FR 0450642**
**01.09.2004 FR 0409259**

(43) Date de publication de la demande:
**05.10.2005 Bulletin 2005/40**

(73) Titulaire: **L'Oréal**
**75008 Paris (FR)**

(72) Inventeurs:
• **Livoreil, Aude**
**75006, PARIS (FR)**
• **Vic, Gabin**
**60280, VENETTE (FR)**
• **Samain, Henri**
**91570, BIEVRES (FR)**

(74) Mandataire: **Martin-Charbonneau, Virginie et al**
**Casalonga & Associés**
**8, avenue Percier**
**75008 Paris (FR)**

(56) Documents cités:
**WO-A-02/058651    FR-A- 2 769 500**
**FR-A- 2 828 992    US-A- 5 184 630**

**Description**

La présente invention a pour objet une composition cosmétique réductrice, pour le premier temps d'une opération de déformation permanente des cheveux comprenant un agent réducteur, et au moins un matériau modifié par incorporation d'une espèce oxydante tel que décrit dans la présente revendication 1. Elle vise également un procédé de déformation permanente des cheveux mettant en oeuvre de tels matériaux modifiés.

La technique la plus usuelle pour obtenir une déformation permanente des cheveux consiste, dans un premier temps à réaliser l'ouverture des liaisons disulfures de la kératine (cystine) à l'aide d'une composition contenant un agent réducteur, puis après avoir de préférence rincé les cheveux, à reconstituer dans un second temps les dites liaisons disulfures en appliquant sur les cheveux préalablement lissés ou mis sous tension par des moyens adéquats tels que des bigoudis, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Ces technologies courantes de transformation de la forme de cheveux, notamment, les compositions de permanente ou de lissage des cheveux, à base de produits thiolés, conduisent à des performances de mise en forme satisfaisantes, mais confèrent une odeur désagréable aux cheveux.

Pour remédier à cet inconvénient, une première solution, mise en oeuvre dans les compositions classiques, consiste à masquer les odeurs par addition de parfum. Cette solution n'est cependant pas satisfaisante car elle ne supprime pas les mauvaises odeurs.

Des solutions alternatives ont été proposées. Par exemple, la demande de brevet US 5,184,630 propose d'ajouter une zéolite à une composition cosmétique, pour réduire les mauvaises odeurs.

Des compositions oxydantes, ou fixateurs utilisant du manganèse, ont également été proposées en remplacement de l'eau oxygénée utilisée habituellement, pour réduire les odeurs. Mais, lors d'une succession permanente/coloration, ces compositions induisent une perturbation de la montée de couleur.

Il existe donc un besoin pour de nouvelles compositions cosmétiques destinées à une opération de déformation permanente des cheveux possédant une grande capacité de diminution des mauvaise odeurs, et qui n'induisent pas de perturbation de la montée de couleur lors d'une succession permanente/coloration.

Après diverses études, la demanderesse a maintenant découvert de façon tout à fait surprenante et inattendue que l'utilisation dans une composition, de zéolites ou zéotypes modifié(e)s par addition de métal tel(le)s que décrit(e)s dans la présente revendication 1 conduit à une diminution importante des mauvaises odeurs sans pour autant induire une perturbation de la montée de couleur, dans le cas d'une succession permanente/coloration et permet d'obtenir une frisure satisfaisante en intensité et en tenue et également dans certains cas, une dégradation moins importante du cheveu par rapport aux compositions précédentes.

La présente invention a donc pour objet une composition aqueuse réductrice non colorante, pour la déformation permanente des cheveux, comprenant, dans un milieu cosmétiquement acceptable :

(i) au moins un agent réducteur de formule :

$$(H)_{m'} \, X \, (R)_n \qquad (I),$$

dans laquelle X représente P, S ou $SO_2$, m' vaut 0 ou 1, n vaut 1, 2 ou 3 et R est un radical $(C_1\text{-}C_{20})$hydrocarboné, linéaire, ramifié, saturé ou insaturé, éventuellement interrompu par un hétéroatome, et comportant éventuellement des substituants choisis parmi un groupement hydroxy, un groupement halogéné, un groupement amine ou un groupement carboxy, un groupement $((C_1\text{-}C_{30})$ alcoxy)carbonyle, un groupement amido, un groupement $((C_1\text{-}C_{30})$ alkyl)amino carbonyle, un groupement $(C_1\text{-}C_{30})$acyle) amino, un groupement mono ou di$((C_1\text{-}C_{30})$alkyl)amino, un groupement mono ou dihydroxy$((C_1\text{-}C_{30})$alkyl)amino,
ou l'un de ses sels en combinaison avec une base, et
(ii) au moins une zéolithe ou zéotype, formant un réseau tridimensionnel répétitif capable d'emprisonner un ou plusieurs ions ou complexes métalliques, modifié par incorporation d'au moins une espèce oxydante choisie parmi les métaux de transition, sous forme de sel, d'oxyde ou de complexes avec un ligand.

On entend par zéotypes, des structures cristallines de type zéolite dans lesquelles tout ou partie des atomes de silicium, d'aluminium ou de phosphore sont remplacés par un ou plusieurs atomes des éléments des colonnes 2 à 13 du tableau périodique, en particulier par des atomes de bore, gallium, fer, chrome, germanium, titane, vanadium, manganèse, cobalt, zinc, béryllium, cuivre, nickel, cobalt, molybdène, ruthénium, rhénium, palladium, argent.

Un zéotype donné peut contenir plusieurs éléments de substitution.

De préférence, la zéolite modifiée est une zéolite naturelle ou artificielle répondant à la formule générale :

$$XM'_{2/n}O.Al_2O_3.YSiO_2.ZH_2O \qquad (II)$$

Dans laquelle, M' est un ion échangeable, de préférence un ion métal monovalent ou divalent, n est la valence de l'ion M', X est un oxyde métallique, Y le nombre de molécules de silice, et Z est le nombre de molécules d'eau de cristallisation.

De préférence, M' représente Na, K, Ca, Mg ou Ba.

La zéolite modifiée est choisie préférentiellement parmi une zéolite de type A, K, L, P-L, O, T, X, Y et Ω, les zéolites à fort taux de silice, la sodalite, la mordenite, l'analcime, la crynoptyrolite, la chabazite, et l'érionite.

De préférence, la zéolite modifiée est une zéolite synthétique.

De préférence, le ou les matériaux modifiés tels que définis ci-dessus et en particulier les zéolites, sont présents à une teneur comprise entre 0,1 et 10%, de préférence entre 1 et 5%, en poids par rapport au poids total de la composition réductrice.

La zéolite peut être modifiée par incorporation d'un métal sous forme de sel de formule :

$$M(S)_n \qquad (II),$$

dans laquelle M est un métal choisi parmi Ti, V, Cr, Mn, Fe, Co, Ni, Cu, Zn, Mo, Ru, Rh, Pd, et Ag ; S est un anion inorganique choisi parmi les ions sulfate, sulfonate, phosphate, phosphonate, chlorure, bromure, et nitrate ; ou un anion organique choisi parmi les ions lactate, citrate, acétate, et dans laquelle n vaut 1 à 8.

Alternativement, la zéolite est modifiée par incorporation d'un métal sous forme d'oxyde de formule :

$$M_{n'}(O)_n \qquad (III),$$

dans laquelle M est un métal choisi parmi Ti, V, Cr, Mn, Fe, Co, Ni, Cu, Zn, Mo, Ru, Rh, Pd, et Ag ; n' vaut 1 à 5, et O représente l'oxygène et n vaut 1 à 8.

La zéolite peut être modifiée par incorporation d'un métal sous forme de complexe avec un ligand, ledit complexe répondant à la formule :

$$M(L)_n \qquad (IV),$$

dans laquelle M est un métal choisi parmi Ti, V, Cr, Mn, Fe, Co, Ni, Cu, Zn, Mo, Ru, Rh, Pd, et Ag ; L est un ligand organique choisi parmi les composés cycliques ou acycliques, saturés ou insaturés, comprenant des groupes carboxylates, des amines primaires, secondaires et tertiaires, des groupes hydroxy, ou un ligand inorganique choisi parmi $NH_3$, NO, et dans laquelle n vaut 1 à 10.

De préférence, L est un ligand organique choisi parmi pyridine, pyridazine, pyrimidine, pyrazine, imidazol, pyrazol, triazol, 2,2'-bispyridylamine, tris-(2-pyridylméthyl)amine, 1,4,7-triazaclyclononane, 1,4,7-triméthyl-1,4,7-triazaclyclononane, 1,5,9-triméthyl-1,5,9-triazacyclododécane, ((bis-(1-méthylimidazol-2-yl)-méthyl))-(2-pyridylméthyl)-amine, N,N'-((bis-(1-méthylimidazol-2-yl)-méthyl))-éthylènediamine, N-bis-(2-benzimidazolylméthyl)-aminoéthanol, 2,6-bis-(bis-(2-benzimidazolylméthyl)aminométhyl)-4-méthylphénol, N,N,N',N'-tetrakis-(2-benzimidazolylméthyl)-2-hydroxy-1,3-diaminopropane, 2,6-bis(bis-(2-pyridylméthyl)aminométhyl)-4-méthylphénol, 1,3-bis(bis-(2-benzimidazolylméthyl)aminométhyl)-benzol, sorbitol, mannitol, érythritol, adonitol, inositol, lactose, et éventuellement leurs sels.

De préférence, le ligand organique est choisi parmi : le 1,4,8 ;11-tetraazacyclotetradécane, le 1,8-diazabicyclo [5.4.0]undec-7-ène, l'éthylènediamine, l'acide nitrilotriacétique, la tris(aminoéthyl)amine, le tris(aminométhyl)méthane, le 1,3,5-triaminocyclohexane, la pyridine-2,6-dicarboxylique, l'acide pyridine-2,6-dicarboxylique, la 2,2',6',2"-terpyridine, la 1,10-phénanthroline, la tris(2-pyridylmethyl)amine, la tris(2-pyridyléthyl)amine, et la N-carboxyméthyl-N-(2-pyridylméthyl)glycine.

La composition réductrice selon l'invention contient un ou plusieurs autres agents réducteurs de formule (I) connus. Parmi ceux-ci, on peut citer l'acide thioglycolique ou l'acide thiolactique et leurs dérivés esters et amides, notamment le monothioglycolate de glycérol, la cystéamine et ses dérivés ($C_1$-$C_4$) acylés tels que la N-acétyl-cystéamine ou la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides tels que ceux décrits dans la demande de brevet EP-A-354 835, les N-mono ou N,N-dialkylmercapto-4-butyramides tels que ceux décrits dans la demande de brevet EP-A-368 763, les aminomercaptoalkylamides, tels que ceux décrits dans la demande de brevet EP-A-432 000, les dérivés des acides N-(mercaptoalkyl)succinamiques et des N-(mercaptoalkyl)succinimides tels que ceux décrits dans la demande de brevet EP-A-465 342, les alkylamino mercaptoalkylamides tels que ceux décrits dans la demande de brevet EP-A-514 282, le mélange azéotrope de thioglycolate de 2-hydroxypropyle et de thioglycolate de (2-hydroxy-1-méthyl) éthyle décrits dans la demande de brevet FR-A-2 679 448, les mercaptoalkylaminoamides tels que ceux décrits dans la demande de brevet FR-A-2 692 481, les N-mercaptoalkylalcanediamides décrits dans la demande de brevet EP-A-653 202, ainsi que les dérivés d'acide formamidine sulfinique. Le cas échéant, ces composés peuvent être utilisés sous forme de sels.

Par non colorante, on entend au sens de la présente invention une composition ne contenant ni colorants directs, ni colorants d'oxydation susceptible de conférer aux fibres après rinçage et séchage une coloration visible en une application. En d'autres termes, l'écart de couleur ΔE, entre fibre traitée et fibre non traitée est inférieur à 5 dans le système L*a*b* avec $\Delta E = (\Delta L^{*2} + \Delta a^{*2} + \Delta b^{*2})^{1/2}$.

Dans un mode de réalisation préféré, la composition réductrice selon l'invention contient également au moins un agent tensioactif de type non-ionique, anionique, cationique ou amphotère. Parmi ceux-ci, on peut citer les alkyl sulfates, les alkyl benzènesulfates, les alkyl éthersulfates, les alkyl sulfonates, les sels d'ammonium quaternaire, les alkyl bétaïnes, les alkyl phénols oxyéthylénés, les alcanolamides d'acides gras, les esters d'acides gras oxyéthylénés ainsi que d'autres tensioactifs non-ioniques du type hydroxypropyléthers.

Lorsque la composition réductrice selon l'invention contient un ou plusieurs agents tensioactifs, celui-ci ou ceux-ci représentent généralement au plus 30 % en poids, et de préférence entre 0,5 et 10 % en poids, par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les brevets français FR2598613 et FR 2 470 596. On peut également utiliser comme agents traitants des silicones volatiles ou non, linéaires ou cycliques, et leurs mélanges, les polydiméthylsiloxanes, les polyorganosiloxanes quaternisés tels que ceux décrits dans la demande de brevet français FR2535730, les polyorganosiloxanes à groupements aminoalkyles modifiés par des groupements alcoxycarbonylalkyles tels que ceux décrits dans le brevet US 4,749,732, des polyorganosiloxanes tels que le copolymère polydiméthylsiloxane-polyoxyalkyle du type diméthicone copolyol, un polydiméthylsiloxane à groupements terminaux stéaroxy (stéaroxy-diméthicone), un copolymère polydiméthylsiloxane dialkylammonium acétate ou un copolymère polydiméthyl-siloxane polyalkylbétaïne décrits dans le brevet britannique n° 2 197 352, des polysiloxanes organo modifiés par des groupements mercapto ou mercaptoalkyles tels que ceux décrits dans le brevet français FR 1 530 369 et dans la demande de brevet européen EP 295 780, ainsi que des silanes tels que le stéaroxytriméthylsilane.

La composition réductrice selon l'invention peut également contenir un ou plusieurs autres ingrédients traitants tels que des polymères cationiques tels que ceux utilisés dans les compositions des brevets français n° 79.32078 (FR 2 472 382) et n° 80.26421 (FR 2 495 931), et en particulier des polymères cationiques du type ionène ou des cyclopolymères, des aminoacides basiques tels que la lysine ou l'arginine, des aminoacides, acides tels que l'acide glutamique ou l'acide aspartique, des peptides et leurs dérivés, des hydrolysats de protéines ou des cires.

La composition réductrice selon l'invention peut également contenir des agents de gonflement et de pénétration, ou des agents permettant de renforcer l'efficacité du réducteur tels que le mélange $SiO_2$/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en $C_3$-$C_6$ tels que par exemple le propanediol-1,2, le propanediol-1,3 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

La composition réductrice selon l'invention peut contenir un solvant tel que par exemple de l'éthanol, du propanol ou de l'isopropanol ou encore du glycérol, à une concentration maximale de 20 % en poids par rapport au poids total de la composition.

En particulier, la composition selon l'invention peut comprendre de l'eau ou un mélange d'eau et d'un ou plusieurs alcools inférieurs en $C_1$-$C_6$, de préférence l'éthanol, l'isopropanol ou le t-butanol

De préférence, la composition réductrice selon l'invention se présente essentiellement sous forme aqueuse, notamment sous la forme d'une lotion épaissie ou non, d'une crème épaisse ou non, ou d'un gel.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables ou d'alcools gras.

On peut également utiliser des liquides ou des gels contenant des agents épaississants tels que des polymères ou des copolymères carboxyvinyliques qui "collent" les cheveux et les maintiennent dans la position lisse pendant le temps de pose.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

Les zéolites modifiées par incorporation d'un métal ou d'un sel de métal sont généralement préparées suivant le mode opératoire décrit ci-dessous.

Une zéolite modifiée selon l'invention est préparée en mettant en contact ladite zéolite avec une solution aqueuse comprenant un ion métallique. Ce contact provoque la substitution entre l'ion échangeable de la zéolite, avec l'ion présent dans la solution aqueuse. Cette mise en contact peut être réalisée à une température comprise entre 10 et 70°C, de préférence entre 40°C, et 60°C, pendant 10 à 24 heures. Le pH de la solution aqueuse est ajusté entre 5 et 7. Cet ajustement permet de prévenir la précipitation des oxydes de métaux à la surface de la zéolite. Les ions métalliques dans la solution aqueuse sont de préférence introduits sous forme de sel de métal. A titre d'exemple, les ions argent utilisés peuvent être sous forme de nitrate d'argent, sulfate d'argent, ou encore acétate d'argent.

Le contenu en métal de la zéolite peut être aisément ajusté par l'homme du métier, par modification de la concentration en métal dans la solution aqueuse.

Une zéolite modifiée, convenant par exemple à la réalisation de l'invention est la zéolite commercialisée par ATO-CECA-DAFA sous le nom commercial ZEOSTOP X.

L'invention a également pour objet une composition aqueuse non réductrice, comprenant au moins un agent réducteur et au moins un matériau solide organique sous forme de zéotype.

De préférence, le matériau solide est un zéotype comportant une espèce oxydante faisant partie intégrante de son réseau tridimensionnel. En particulier, l'espèce oxydante peut être un métal choisi parmi Ti, V, Cr, Mn, Fe, Co, Ni, Cu, Zn, Mo, Ru, Rh, Pd, et Ag.

Le métal peut être sous forme de complexe avec un ligand, ledit complexe répondant à la formule :

$$M(L)_n \qquad (IV),$$

dans laquelle M est un métal choisi parmi Ti, V, Cr, Mn, Fe, Co, Ni, Cu, Zn, Mo, Ru, Rh, Pd, et Ag ; L est un ligand organique choisi parmi les composés cycliques ou acycliques, saturés ou insaturés, comprenant des groupes carboxylates, des amines primaires, secondaires et tertiaires, des groupes hydroxy, ou un ligand inorganique choisi parmi $NH_3$, NO, et dans laquelle n vaut 1 à 10.

De préférence, le ligand organique est choisi parmi : le 1,4,8 ;11-tetraazacyclotetradécane, le 1,8-diazabicyclo [5.4.0]undec-7-ène, l'éthylènediamine, l'acide nitrilotriacétique, la tris(aminoéthyl)amine, le tris(aminométhyl)méthane, le 1,3,5-triaminocyclohexane, la pyridine-2,6-dicarboxylique, l'acide pyridine-2,6-dicarboxylique, la 2,2',6',2"-terpyridine, la 1,10-phénanthroline, la tris(2-pyridylmethyl)amine, la tris(2-pyridyléthyl)amine, et la N-carboxyméthyl-N-(2-pyridylméthyl)glycine.

Des procédés de préparation de zéotypes, au titane et au fer, convenant pour la réalisation de la présente invention, sont décrits respectivement dans les demandes de brevets US4,480,135 et US5,110,995.

En particulier, la demande de brevet US4,480,135 décrit la préparation d'une silicalite de titane (TS-1), à partir d'un malange réactionel comprenant un oxyde de silice, un oxyde de titane, éventuellement un oxyde alcalin, une base organique azotée et de l'eau.

La source d'oxyde de silice peut être un tetraalkylorthosilicate, un silicate sous forme colloïdale, ou encore un silicate de métal alcalin, par exemple Na, ou K.

La source d'oxyde de titane est un composé hydrolysable par exemple, $TiCl_4$, ou $TiOCl_2$ $Ti(alcoxy)_4$, et de préférence, $Ti(OC_2H_5)_4$. La base organique peut être un hydroxyde de tetraalkylammonium, et en particulier, l'hydroxyde de tetrapropylammonium.

Le mélange réactionnel est soumis à un traitement hydrothermique dans un autoclave, à une température comprise entre 130°C et 200°C, sous sa propre pression, pendant 6 à 30 jours, jusqu'à ce que des cristaux de précurseurs de TS-1 se développent. Les cristaux sont séparés de la solution mère, lavés avec précaution, et séchés. A l'état anhydre, les cristaux ont la composition suivante :

$$xTiO_2(1-x)SiO_2\, 0,04(RN+)_2O$$

où x vaut 0,0001 à 0,04

Les cristaux précurseurs sont chauffés 1 à 72 heures dans l'air à 550°C pour éliminer complètement la base organique azotée. La silicalite de titane finale a la composition suivante :

$$xTiO_2\, (1-x)SiO_2$$

La demande de brevet US5,110,995 décrit la préparation d'un zéotype au fer, à partir d'un mélange comprenant une source de silice, une source de de fer, et si nécessaire, une source de $El^{n+}$ ( où El est un élément des Périodes 2, 3, 4 ou 5 de la classification périodique des éléments), une base, un tensio actif organique, et éventuellement un germe de cristallisation.

Le mélange réactionnel est soumis à un traitement hydrothermique dans un autoclave, pendant 1 à 30 jours, à une température comprise entre 80°C et 200°C. Lorsque la cristallisation est complète, les cristaux sont filtrés, lavés à l'eau distillée, et séchés. Le produit est enfin calciné à une tempérautre comprise entre 520°C et 550°C de manière à éliminer les inclusions organiques. Si nécessaire, une décationisation peut être réalisée par une solution de $NH_4OH$ + $NH_4Cl$ ou une solution d'acides inorganiques. Si nécessaire, un élément, peut être introduit dans la matrice Fe-Si en utilisant une méthode par échange d'ions, ou par imprégnation.

L'invention concerne encore un procédé de déformation permanente des cheveux mettant en oeuvre une composition réductrice comprenant au moins un agent réducteur et une zéolithe ou zéotype tel(le) que décrit(e) dans la présente revendication 1.

Le procédé de déformation permanente des cheveux selon l'invention comprend :

- une étape d'application sur les cheveux d'une composition réductrice selon l'invention, pour réduire les liaisons disulfures de la kératine ;
- une étape de fixation par oxydation, pour refermer lesdites liaisons, par application d'une composition oxydante sur les cheveux ou par mise en contact des cheveux avec l'oxygène de l'air.

De préférence, la zéolithe ou zéotype tel(le) que décrit(e) dans la présente revendication 1 est incorporée dans la composition réductrice au moment de l'emploi.

Alternativement, la zéolithe ou zéotype tel(le) que décrit(e) dans la présente revendication 1 est déposée sur les cheveux avant l'application d'une composition réductrice. Ainsi, l'invention concerne également un procédé de déformation permanente des cheveux comprenant :

- Une étape d'application sur les cheveux d'une composition aqueuse comprenant, dans un milieu cosmétiquement acceptable au moins une zéolithe ou zéotype tel(le) que décrit(e) dans la présente revendication 1 formant un réseau tridimensionnel répétitif capable d'emprisonner un ou plusieurs ions ou complexes métalliques, modifié par incorporation d'au moins une espèce oxydante choisie parmi les métaux de transition, sous forme de sel, d'oxyde ou de complexes avec un ligand,
- une étape d'application sur les cheveux d'une composition aqueuse réductrice, pour réduire les liaisons disulfures de la kératine, comprenant, dans un milieu cosmétiquement acceptable au moins un agent réducteur de formule :

$$(H)_{m'}X(R)_n \qquad (I),$$

dans laquelle X représente P, S ou $SO_2$, m' vaut 0 ou 1, n vaut 1, 2 ou 3 et R est un radical $(C_1\text{-}C_{20})$hydrocarboné, linéaire, ramifié, saturé ou insaturé, éventuellement interrompu par un hétéroatome, et comportant éventuellement des substituants choisis parmi un groupement hydroxy, un groupement halogéné, un groupement amine ou un groupement carboxy, un groupement $((C_1\text{-}C_{30})$ alcoxy)carbonyle, un groupement amido, un groupement $((C_1\text{-}C_{30})$ alkyl)amino carbonyle, un groupement $(C_1\text{-}C_{30})$acyle) amino, un groupement mono ou di$((C_1\text{-}C_{30})$alkyl)amino, un groupement mono ou dihydroxy$((C_1\text{-}C_{30})$alkyl)amino, ou l'un de ses sels en combinaison avec une base, et

- une étape de fixation par oxydation, pour reformer lesdites liaisons dissulfures, par application d'une composition oxydante sur les cheveux ou par mise en contact des cheveux avec l'oxygène de l'air.

Les cheveux sont mis en forme en utilisant des moyens mécaniques bien connus de l'homme de l'art.

Lorsque l'on souhaite réaliser une permanente, on utilise de préférence des bigoudis, la composition réductrice étant appliquée avant ou après les moyens de mise en forme des cheveux, et la composition oxydante de fixation étant appliquée après la composition réductrice, avec ou sans étape intermédiaire ou subséquente de rinçage ou d'application de composition intermédiaire.

De préférence, on applique une composition réductrice selon l'invention sur des cheveux mouillés préalablement enroulés sur des rouleaux ayant de 2 à 30 mm de diamètre. La composition peut également être appliquée au fur et à mesure de l'enroulage des cheveux. Généralement, on laisse ensuite agir la composition réductrice pendant un temps de 5 à 60 minutes, de préférence de 15 à 45 minutes, puis on rince abondamment. On applique alors, sur les cheveux enroulés, la composition oxydante permettant de reformer les liaisons disulfures de la kératine, pendant un temps de pose de 2 à 10 minutes. Après avoir enlevé les rouleaux, on rince abondamment la chevelure.

On peut également, après application de la composition réductrice, soumettre la chevelure à un traitement thermique par chauffage à une température comprise entre 30 et 60°C pendant tout ou partie du temps de pose. Dans la pratique, cette opération peut être conduite au moyen d'un casque de coiffure, d'un sèche cheveux, d'un générateur de rayons infrarouges et d'autres appareils chauffants classiques.

On peut notamment utiliser, à la fois comme moyen de chauffage et de mise en forme de la chevelure, un fer chauffant à une température comprise entre 60 et 250°C et de préférence entre 120 et 220°C, l'utilisation du fer chauffant se faisant entre l'étape d'application de la composition réductrice et l'étape de fixation.

Lorsque l'on souhaite effectuer le défrisage ou le décrêpage des cheveux, on applique sur les cheveux une composition réductrice selon l'invention, puis l'on soumet les cheveux à une déformation mécanique permettant de les fixer dans leur nouvelle forme, par une opération de lissage des cheveux avec un peigne à larges dents, avec le dos d'un peigne ou à la main. Après généralement un temps de pose de 5 à 60 minutes, de préférence de 15 à 45 minutes, on procède à un nouveau lissage, puis on rince soigneusement et on applique la composition oxydante telle que définie ci-dessus, qu'on laisse agir pendant environ 2 à 10 minutes, puis on rince abondamment les cheveux.

La composition oxydante peut être toute composition oxydante couramment utilisée pour la déformation permanente des cheveux et contient un agent oxydant choisi de préférence parmi l'eau oxygénée, les bromates alcalins, les persels, les polythionates et un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 %, en poids par rapport au poids total de la composition oxydante.

Le pH de la composition oxydante est généralement compris entre 2 et 10.

Comme expliqué précédemment, l'application de la composition oxydante peut être effectuée immédiatement ou être différée.

Selon un mode de réalisation particulier des procédés selon l'invention, le lissage des cheveux peut également être effectué, en tout ou partie, à l'aide d'un fer chauffant entre 60 et 250°C, et de préférence entre 120 et 220°C.

L'invention concerne également un kit, notamment pour la déformation permanente des cheveux comprenant, dans un premier compartiment, en tant que composition réductrice, une composition réductrice définie dans la présente revendication 1, comprenant au moins **une zéolithe ou zéotype**, formant un réseau tridimensionnel répétitif capable d'emprisonner un ou plusieurs ions ou complexes métalliques, modifié par incorporation d'au moins une espèce oxydante choisie parmi les métaux de transition, sous forme de sel, d'oxyde ou de complexes avec un ligand. et dans un second compartiment, une composition oxydante.

Généralement, la composition oxydante comprend au moins un agent oxydant choisi parmi l'eau oxygénée, les bromates alcalins, les persels, les polythionates et un mélange de bromate alcalin et de persel.

L'invention concerne également l'utilisation d'au moins **une zéolithe ou zéotype**, formant un réseau tridimensionnel répétitif capable d'emprisonner un ou plusieurs ions ou complexes métalliques, modifié par incorporation d'au moins une espèce oxydante choisie parmi les métaux de transition, sous forme de sel, d'oxyde ou de complexes avec un ligand, pour réduire les problèmes d'odeur liés aux procédés de déformation permanente des cheveux.

L'invention pourra être mieux comprise à l'aide des exemples non limitatifs qui suivent et qui constituent des modes de réalisation préférentiels des compositions selon l'invention.

## Exemple 1 : Exemple de composition

On a préparé une composition réductrice de déformation permanente des cheveux « témoin » en procédant au mélange des composés suivants :

## A1

- Acide thioglycolique :        9g
- Ammoniaque à 20% :         qsp pH 9
- Eau déminéralisée :        qsp 100 g

On a préparé également une composition réductrice de déformation permanente des cheveux selon l'invention en procédant au mélange des composés suivants :

## A2

- Acide thioglycolique :        9g
- ZEOSTOP $X_{(1)}$ :        5g
- ammoniaque à 20% :         qsp pH 9
- eau déminéralisée :        qsp 100 g

(1) zéolite ZEOSTOP X commercialisée par CECA, comprenant Cu, ZN, et Ag.

On a préparé enfin la composition oxydante suivante :

**B**

- Eau oxygénée : 8 vol
- Acide citrique qsp pH 3
- Eau déminéralisée qsp 100g

Des cheveux ont été lavés, essorés et enroulés sur des bigoudis. On applique sur les cheveux la composition réductrice A1 ou A2. On a laissé agir la composition A1 ou A2 pendant environ 15 minutes, à température ambiante, sous film plastique.

On a retiré le film plastique et l'odeur est évaluée. Puis la composition a été rincée à l'eau et la composition B de fixation a été appliquée, suivie d'une pause de 5 minutes à température ambiante.

La composition est rincée, les bigoudis défaits et on a évalué la frisure.

On a constaté que pour les cheveux traités avec la composition A1 puis B, l'odeur désagréable est beaucoup plus forte que pour les cheveux traités avec la composition traités avec la composition A2 puis B, pour une frisure équivalente.

**Revendications**

1. Composition aqueuse réductrice non colorante, pour la déformation permanente des cheveux, comprenant, dans un milieu cosmétiquement acceptable :

   (i) au moins un agent réducteur de formule :

   $$(H)_{m'} \, X \, (R)_n \qquad (I),$$

   dans laquelle X représente P, S ou $SO_2$, m' vaut 0 ou 1, n vaut 1, 2 ou 3 et R est un radical $(C_1\text{-}C_{20})$hydrocarboné, linéaire, ramifié, saturé ou insaturé, éventuellement interrompu par un hétéroatome, et comportant éventuellement des substituants choisis parmi un groupement hydroxy, un groupement halogéné, un groupement aminé ou un groupement carboxy, un groupement $((C_1\text{-}C_{30})$ alcoxy)carbonyle, un groupement amido, un groupement $((C_1\text{-}C_{30})$alkyl)amino carbonyle, un groupement $(C_1\text{-}C_{30})$acyle) amino, un groupement mono ou di$((C_1\text{-}C_{30})$ alkyl)amino, un groupement mono ou dihydroxy$((C_1\text{-}C_{30})$alkyl)amino, ou l'un de ses sels en combinaison avec une base, et

   (ii) au moins une zéolite ou zéotype, formant un réseau tridimensionnel répétitif capable d'emprisonner un ou plusieurs ions ou complexes métalliques, modifié par incorporation d'au moins une espèce oxydante choisie parmi les métaux de transition, sous forme de sel, d'oxyde ou de complexes avec un ligand.

2. Composition selon la revendication 1, **caractérisée en ce que** la zéolite modifiée est une zéolite naturelle ou artificielle répondant à la formule générale :

   $$XM'_{2/n}O.Al_2O_3.YSiO_2.ZH_2O \qquad (II)$$

   Dans laquelle, M' est un ion échangeable, de préférence un ion métal monovalent ou divalent, n est la valence de l'ion M', X est un oxyde métallique, Y le nombre de molécules de silice, et Z est le nombre de molécules d'eau de cristallisation.

3. Composition selon la revendication 1 ou 2, **caractérisée en ce que** la zéolite modifiée est choisie parmi une zéolite de type A, K, L, P-L, O, T, X, Y et Q, les zéolites à fort taux de silice, la sodalite, la mordenite, l'analcime, la crynoptyrolite, la chabazite, et l'érionite.

4. Composition selon la revendication 1 ou 2, **caractérisée en ce que** la zéolite modifiée est une zéolite synthétique.

**5.** Composition selon l'une quelconque des revendications 1 à 4, **caractérisée en ce que** M' représente Na, K, Ca, Mg ou Ba.

**6.** Composition selon l'une quelconque des revendications 1 à 5, **caractérisée en ce que** la zéolite est modifiée par incorporation d'un métal sous forme de sel, de formule :

$$M(S)_n \qquad (II),$$

dans laquelle M est un métal choisi parmi Ti, V, Cr, Mn, Fe, Co, Ni, Cu, Zn, Mo, Ru, Rh, Pd, et Ag ; S est un anion inorganique choisi parmi les ions sulfate, sulfonate, phosphate, phosphonate, chlorure, bromure, et nitrate ; ou un anion organique choisi parmi les ions lactate, citrate, acétate, et dans laquelle n vaut 1 à 8.

**7.** Composition selon l'une quelconque des revendications 1 à 5, **caractérisée en ce que** la zéolite est modifiée par incorporation d'un métal sous forme d'oxyde de formule :

$$M_{n'}(O)_n \qquad (III),$$

dans laquelle M est un métal choisi parmi Ti, V, Cr, Mn, Fe, Co, Ni, Cu, Zn, Mo, Ru, Rh, Pd, et Ag ; n' vaut 1 à 5, et O représente l'oxygène et n vaut 1 à 8.

**8.** Composition selon l'une quelconque des revendications 1 à 5, **caractérisée en ce que** la zéolite est modifiée par incorporation d'un métal sous forme de complexe avec un ligand, ledit complexe répondant à la formule :

$$M(L)_n \qquad (IV),$$

dans laquelle M est un métal choisi parmi Ti, V, Cr, Mn, Fe, Co, Ni, Cu, Zn, Mo, Ru, Rh, Pd, et Ag ; L est un ligand organique choisi parmi les composés cycliques ou acycliques, saturés ou insaturés, comprenant des groupes carboxylates, des amines primaires, secondaires et tertiaires, des groupes hydroxy, ou un ligand inorganique choisi parmi $NH_3$, NO, et dans laquelle n vaut 1 à 10.

**9.** Composition selon la revendication 8, **caractérisée en ce que** le ligand organique est choisi parmi : le 1,4,8 11-tetraazacyclotetradécane, le 1,8-diazabicyclo[5.4.0]undec-7-ène, l'éthylènediamine, l'acide nitrilotriacétique, la tris (aminoéthyl)amine, le tris(aminométhyl)méthane, le 1,3,5-triaminocyclohexane, la pyridine-2,6-dicarboxylique, l'acide pyridine-2,6-dicarboxylique, la 2,2',6',2"-terpyridine, la 1,10-phénanthroline, la tris(2-pyridylmethyl)amine, la tris (2-pyridyléthyl)amine, et la N-carboxyméthyl-N-(2-pyridylméthyl)glycine.

**10.** Composition selon la revendication 1, **caractérisée en ce que** le matériau solide est un zéotype comportant une espèce oxydante faisant partie intégrante de son réseau tridimensionel.

**11.** Composition selon la revendication 10, **caractérisée en ce que** l'espèce oxydante est un métal choisi parmi Ti, V, Cr, Mn, Fe, Co, Ni, Cu, Zn, Mo, Ru, Rh, Pd, et Ag.

**12.** Composition selon la revendication 11, **caractérisée en ce que** le métal est sous forme de complexe avec un ligand, ledit complexe répondant à la formule :

$$M(L)_n \qquad (IV),$$

dans laquelle M est un métal choisi parmi Ti, V, Cr, Mn, Fe, Co, Ni, Cu, Zn, Mo, Ru, Rh, Pd, et Ag ; L est un ligand organique choisi parmi les composés cycliques ou acycliques, saturés ou insaturés, comprenant des groupes carboxylates, des amines primaires, secondaires et tertiaires, des groupes hydroxy, ou un ligand inorganique choisi parmi $NH_3$, NO, et dans laquelle n vaut 1 à 10.

**13.** Composition selon la revendication 12, **caractérisée en ce que** le ligand organique est choisi parmi : le 1,4,8 ;11-tetraazacyclotetradécane, le 1,8-diazabicyclo[5.4.0]undec-7-ène, l'éthylènediamine, l'acide nitrilotriacétique, la tris (aminoéthyl)amine, le tris(aminométhyl)méthane, le 1,3,5-triaminocyclohexane, la pyridine-2,6-dicarboxylique, l'acide pyridine-2,6-dicarboxylique, la 2,2',6',2"-terpyridine, la 1,10-phénanthroline, la tris(2-pyridylmethyl)amine, la tris (2-pyridyléthyl)amine, et la N-carboxyméthyl-N-(2-pyridylméthyl)glycine.

**14.** Composition selon l'une quelconque des revendications 1 à **13**, **caractérisée en ce que** le ou les matériaux solides, sont présents à une teneur comprise entre 0,1 et 10%, de préférence entre 1 et 5%, en poids par rapport au poids total de la composition.

**15.** Composition selon l'une quelconque des revendications 1 à **14**, **caractérisée en ce que** le ou les agents réducteurs sont choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les N-(mercaptoalkyl)-ro-hydroxyalkylamides, les N-mono ou N,N-dialkylmercapto-4-butyramides, les aminomercaptoalkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique, et leurs sels.

**16.** Composition selon l'une quelconque des revendications 1 à **15**, **caractérisée en ce qu'**elle comprend au moins un agent tensioactif choisi parmi les alkyl sulfates, les alkyl benzènesulfates, les alkyl éthersulfates, les alkyl sulfonates, les sels d'ammonium quaternaire, les alkyl bétaïnes, les alkyl phénols oxyéthylénés, les alcanolamides d'acides gras, les esters d'acides gras oxyéthylénés, les hydroxypropyléthers.

**17.** Composition selon la revendication **16**, **caractérisée en ce que** le ou les agents tensioactifs représentent au plus 30% en poids, de préférence entre 0,5 et 10% en poids, du poids total de la composition.

**18.** Composition selon l'une quelconque des revendications 1 à **17**, **caractérisée en ce qu'**elle comprend de l'eau ou un mélange d'eau et d'un ou plusieurs alcools inférieurs en $C_1$-$C_6$, de préférence l'éthanol, l'isopropanol ou le t-butanol.

**19.** Composition selon l'une quelconque des revendications 1 à **18**, **caractérisée en ce qu'**elle se présente sous forme aqueuse, de préférence sous la forme d'une lotion, épaissie ou non, d'une crème, épaissie ou non, ou d'un gel.

**20.** Procédé de déformation permanente des cheveux comprenant :

- une étape d'application sur les cheveux d'une composition réductrice telle que définie dans l'une quelconque des revendications 1 à **18**, pour réduire les liaisons disulfures de la kératine,
- une étape de fixation par oxydation, pour reformer lesdites liaisons, par application d'une composition oxydante sur les cheveux ou par mise en contact des cheveux avec l'oxygène de l'air.

**21.** Procédé selon la revendication **20**, **caractérisé en ce que** le matériau modifié est incorporé dans la composition réductrice au moment de l'emploi.

**22.** Procédé de déformation permanente des cheveux comprenant :

- une étape d'application sur les cheveux d'une composition aqueuse comprenant, dans un milieu cosmétiquement acceptable au moins **une zéolyte ou zéotype**, formant un réseau tridimensionnel répétitif capable d'emprisonner un ou plusieurs ions ou complexes métalliques, modifié par incorporation d'au moins une espèce oxydante choisie parmi les métaux de transition, sous forme de sel, d'oxyde ou de complexes avec un ligand,
- une étape d'application sur les cheveux d'une composition aqueuse réductrice, pour réduire les liaisons disulfures de la kératine, comprenant, dans un milieu cosmétiquement acceptable au moins un agent réducteur de formule :

$$(H)_{m'} \, X \, (R)_n \qquad (I),$$

dans laquelle X représente P, S ou $SO_2$, m' vaut 0 ou 1, n vaut 1, 2 ou 3 et R est un radical ($C_1$-$C_{20}$)hydrocarboné, linéaire, ramifié, saturé ou insaturé, éventuellement interrompu par un hétéroatome, et comportant éventuellement des substituants choisis parmi un groupement hydroxy, un groupement halogéné, un groupement amine ou un groupement carboxy, un groupement (($C_1$-$C_{30}$) alcoxy)carbonyle, un groupement amido, un groupement (($C_1$-$C_{30}$)alkyl)amino carbonyle, un groupement ($C_1$-$C_{30}$)acyle) amino, un groupement mono ou di(($C_1$-$C_{30}$) alkyl)amino, un groupement mono ou dihydroxy(($C_1$-$C_{30}$)alkyl)amino, ou l'un de ses sels en combinaison avec une base, et
- **la zéolite ou zéotype est déposée sur les cheveux avant l'application de la composition réductrice,**

- une étape de fixation par oxydation, pour reformer lesdites liaisons dissulfures, par application d'une composition oxydante sur les cheveux ou par mise en contact des cheveux avec l'oxygène de l'air.

**23.** Procédé selon l'une quelconque des revendications **20 à 22**, **caractérisé en ce que** on laisse agir la composition réductrice pendant 5 à 60 minutes, de préférence pendant 15 à 45 minutes.

**24.** Procédé selon l'une quelconque des revendication **20 à 23**, **caractérisé en ce que** la composition oxydante comprend au moins un agent oxydant choisi parmi l'eau oxygénée, les bromates alcalins, les persels, les polythionates et un mélange de bromate alcalin et de persel.

**25.** Procédé selon l'une quelconque des revendications **20** à **24 caractérisé en ce qu'**il comprend, entre l'étape d'application sur les cheveux de la composition réductrice et l'étape de fixation, une étape de chauffage des cheveux avec un fer chauffant à une température comprise entre 60 et 250°C, de préférence entre 120 et 220°C.

**26.** Kit pour la déformation permanente des cheveux comprenant dans un premier compartiment une composition réductrice telle que définie dans l'une quelconque des revendications 1 à **19**, et dans un second compartiment une composition oxydante.

**27.** Utilisation dans une composition réductrice selon l'une quelconque des revendications 1 à **19**, d'un matériau solide organique, inorganique ou hybride, formant un réseau tridimensionnel répétitif capable d'emprisonner un ou plusieurs ions ou complexes métalliques, modifié par incorporation d'au moins une espèce oxydante choisie parmi les métaux de transition, sous forme de sel, d'oxyde ou de complexes avec un ligand, pour réduire les problèmes d'odeur liés aux procédés de déformation permanente des cheveux, le matériau solide organique, inorganique ou hybride étant choisi parmi une zéolithe ou zéotype.

**Claims**

**1.** Non-colouring reductive aqueous composition, for permanently reshaping the hair, comprising, in a cosmetically acceptable medium:

(i) at least one reducing agent of formula:

$$(H)_{m'}X(R)_n \qquad (I),$$

in which X represents P, S or $SO_2$, m' is equal to 0 or 1, n is equal to 1, 2 or 3 and R is a linear or branched, saturated or unsaturated $C_1$-$C_{20}$ hydrocarbon-based radical, optionally interrupted with a heteroatom, and optionally comprising substituents chosen from a hydroxyl group, a halogenated group, an amine group or a carboxyl group, a (($C_1$-$C_{30}$) alkoxy) carbonyl group, an amido group, a (($C_1$-$C_{30}$)alkyl)aminocarbonyl group, a (($C_1$-$C_{30}$) acyl) amino group, a mono- or di(($C_1$-$C_{30}$)alkyl)amino group and a mono- or dihydroxy(($C_1$-$C_{30}$) alkyl) amino group,
or a salt thereof in combination with a base, and
(ii) **at least one zeolite or zeotype**, forming a repeating three-dimensional network capable of trapping one or more metal ions or complexes, modified by incorporation of at least one oxidizing species chosen from transition metals, in the form of salt, of oxide or of complexes with a ligand.

**2.** Composition according to Claim **1**, **characterized in that** the modified zeolite is a natural or artificial zeolite corresponding to the general formula:

$$X_{M'\ 2/n}O.\ Al_2O_3.\ YSiO_2\ .\ ZH_2O \qquad (II)$$

in which M' is an exchangeable ion, preferably a monovalent or divalent metal ion, n is the valency of the ion M', X is a metal oxide, Y is the number of silica molecules, and Z is the number of molecules of water of crystallization.

**3.** Composition according to Claim **1** or **2**, **characterized in that** the modified zeolite is chosen from a zeolite of the type A, K, L, P-L, O, T, X, Y and $\Omega$, zeolites with a high content of silica, sodalite, mordenite, analcime, crynoptyrolite, chabazite and erionite.

4. Composition according to Claim **1** or **2**, **characterized in that** the modified zeolite is a synthetic zeolite.

5. Composition according to any one of Claims **1** to **4**, **characterized in that** M' represents Na, K, Ca, Mg or Ba.

6. Composition according to any one of Claims **1** to **5**, **characterized in that** the zeolite is modified by incorporation of a metal in salt form, of formula:

$$M(S)_n \qquad (II),$$

in which M is a metal chosen from Ti, V, Cr, Mn, Fe, Co, Ni, Cu, Zn, Mo, Ru, Rh, Pd and Ag; S is an inorganic anion chosen from sulfate, sulfonate, phosphate, phosphonate, chloride, bromide and nitrate ions; or an organic anion chosen from lactate, citrate and acetate ions, and in which n is equal to 1 to 8.

7. Composition according to any one of Claims **1** to **5**, **characterized in that** the zeolite is modified by incorporation of a metal in oxide form of formula:

$$M_{n'}(O)_n \qquad (III),$$

in which M is a metal chosen from Ti, V, Cr, Mn, Fe, Co, Ni, Cu, Zn, Mo, Ru, Rh, Pd and Ag; n' is equal to 1 to 5, and O represents oxygen and n is equal to 1 to 8.

8. Composition according to any one of Claims **1** to **5**, **characterized in that** the zeolite is modified by incorporation of a metal in the form of a complex with a ligand, the said complex corresponding to the formula:

$$M(L)_n \qquad (IV),$$

in which M is a metal chosen from Ti, V, Cr, Mn, Fe, Co, Ni, Cu, Zn, Mo, Ru, Rh, Pd and Ag; L is an organic ligand chosen from cyclic or acyclic, saturated or unsaturated compounds, comprising carboxylate groups, primary, secondary and tertiary amines, hydroxyl groups, or an inorganic ligand chosen from $NH_3$ and NO, and in which n is equal to 1 to 10.

9. Composition according to Claim **8**, **characterized in that** the organic ligand is chosen from: 1,4,8,11-tetraazacyclotetradecane, 1,8-diazobicyclo[5.4.0]undec-7-ene, ethylenediamine, nitrilotriacetic acid, tris(aminoethyl)amine, tris(aminomethyl)methane, 1,3,5-triaminocyclohexane, pyridine-2,6-dicarboxylic acid, 2,2',6',2''-terpyridine, 1,10-phenanthroline, tris(2-pyridylmethyl)amine, tris(2-pyridylethyl)amine and N-carboxymethyl-N-(2-pyridylmethyl)glycine.

10. Composition according to Claim **1**, **characterized in that** the solid material is a zeolite comprising an oxidizing species forming an integral part of its three-dimensional network.

11. Composition according to Claim **10**, **characterized in that** the oxidizing species is a metal chosen from Ti, V, Cr, Mn, Fe, Co, Ni, Cu, Zn, Mo, Ru, Rh, Pd and Ag.

12. Composition according to Claim **11**, **characterized in that** the metal is in the form of a complex with a ligand, the said complex corresponding to the formula:

$$M(L)_n \qquad (IV),$$

in which M is a metal chosen from Ti, V, Cr, Mn, Fe, Co, Ni, Cu, Zn, Mo, Ru, Rh, Pd and Ag; L is an organic ligand chosen from cyclic or acyclic, saturated or unsaturated compounds, comprising carboxylate groups, primary, secondary and tertiary amines, hydroxyl groups, or an inorganic ligand chosen from $NH_3$ and NO, and in which n is equal to 1 to 10.

13. Composition according to Claim **12**, **characterized in that** the organic ligand is chosen from: 1,4,8,11-tetraazacyclotetradecane, 1,8-diazobicyclo[5.4.0]undec-7-ene, ethylenediamine, nitrilotriacetic acid, tris(aminoethyl)amine, tris(aminomethyl)methane, 1,3,5-triaminocyclohexane, pyridine-2,6-dicarboxylic acid, 2,2',6',2''-terpyridine, 1,10-phenanthroline, tris(2-pyridylmethyl)amine, tris(2-pyridylethyl)amine and N-carboxymethyl-N-(2-pyridylmethyl)glycine.

**14.** Composition according to any one of Claims **1** to **13**, **characterized in that** the solid material(s) are present in a content of between 0.1% and 10% and preferably between 1% and 5% by weight relative to the total weight of the composition.

**15.** Composition according to any one of Claims **1** to **14**, **characterized in that** the reducing agent(s) are chosen from thioglycolic acid, thiolactic acid, glyceryl monothioglycolate, cysteamine, N-acetylcysteamine, N-propionylcysteamine, cysteine, N-acetylcysteine, thiomalic acid, pantheteine, 2,3-dimercaptosuccinic acid, N-(mercaptoalkyl)-$\omega$-hydroxyalkylamides, N-mono- or N,N-dialkylmercapto-4-butyramides, aminomercaptoalkylamides, N-(mercaptoalkyl) succinamic acid derivatives and N-(mercaptoalkyl)succinimides, alkylamino mercaptoalkylamides, the azeotropic mixture of 2-hydroxypropyl thioglyconate and of (2-hydroxy-1-methyl)ethyl thioglycolate, mercaptoalkylaminoamides, N-mercaptoalkylalkanediamides and formamidinesulfinic acid derivatives, and salts thereof.

**16.** Composition according to any one of Claims **1** to **15**, **characterized in that** it comprises at least one surfactant chosen from alkyl sulfates, alkyl benzene sulfates, alkyl ether sulfates, alkyl sulfonates, quaternary ammonium salts, alkyl betaines, oxyethylenated alkylphenols, fatty acid alkanolamides, oxyethylenated fatty acid esters and hydroxypropyl ethers.

**17.** Composition according to Claim **16**, **characterized in that** the surfactant(s) represent not more than 30% by weight and preferably between 0.5% and 10% by weight, relative to the total weight of the composition.

**18.** Composition according to any one of Claims **1** to **17**, **characterized in that** it comprises water or a mixture of water and of one or more $C_1$-$C_6$ lower alcohols, preferably ethanol, isopropanol or t-butanol.

**19.** Composition according to any one of Claims **1** to **18**, **characterized in that** it is in aqueous form, preferably in the form of an optionally thickened lotion, an optionally thickened cream, or a gel.

**20.** Process for permanently reshaping the hair, comprising:

- a step of applying to the hair a reducing composition as defined in any one of Claims **1** to **18**, to reduce the disulfide bonds of keratin,
- a step of fixing by oxidation, to reform the said bonds, by applying an oxidizing composition to the hair or by placing the hair in contact with atmospheric oxygen.

**21.** Process according to Claim **20**, **characterized in that** the modified material is incorporated into the reducing composition at the time of use.

**22.** Process for permanently reshaping the hair, comprising:

- a step of applying to the hair an aqueous composition comprising, in a cosmetically acceptable medium, at least one **zeolite or zeotype**, forming a repeating three-dimensional network capable of trapping one or more metal ions or complexes, modified by incorporation of at least one oxidizing species chosen from transition metals, in the form of salt, of oxide or of complexes with a ligand,
- a step of applying to the hair a reductive aqueous composition, to reduce the disulfide bonds of keratin, comprising, in a cosmetically acceptable medium, at least one reducing agent of formula:

$$(H)_{m'}X(R)_n \qquad (I),$$

in which X represents P, S or $SO_2$, m' is equal to 0 or 1, n is equal to 1, 2 or 3 and R is a linear or branched, saturated or unsaturated $C_1$-$C_{20}$ hydrocarbon-based radical, optionally interrupted with a heteroatom, and optionally comprising substituents chosen from a hydroxyl group, a halogenated group, an amine group or a carboxyl group, a (($C_1$-$C_{30}$) alkoxy) carbonyl group, an amido group, a (($C_1$-$C_{30}$) alkyl) aminocarbonyl group, a (($C_1$-$C_{30}$) acyl) amino group, a mono- or di(($C_1$-$C_{30}$)alkyl)amino group and a mono- or dihydroxy(($C_1$-$C_{30}$) alkyl)amino group, or a salt thereof in combination with a base, and
- **the zeolite or zeotype is deposited on the hair before applying the reducing composition,**
- a step of fixing by oxidation, to reform the said disulfide bonds, by applying an oxidizing composition to the hair or by placing the hair in contact with atmospheric oxygen.

**23.** Process according to any one of Claims **20** to **22**, **characterized in that** the reducing composition is left to act for 5 to 60 minutes and preferably for 15 to 45 minutes.

24. Process according to any one of Claims **20** to **23**, **characterized in that** the oxidizing composition comprises at least one oxidizing agent chosen from aqueous hydrogen peroxide solution, alkali metal bromates, persalts, polythionates and a mixture of alkali metal bromate and of persalt.

25. Process according to any one of Claims **20** to **24**, **characterized in that** it comprises, between the step of applying the reducing composition to the hair and the fixing step, a step of heating the hair with a heating iron to a temperature of between 60 and 250°C and preferably between 120 and 220°C.

26. Kit for permanently reshaping the hair, comprising, in a first compartment, a reducing composition as defined in any one of Claims **1** to **19**, and, in a second compartment, an oxidizing composition.

27. Use, in a reducing composition according to any one of Claims **1** to **19**, of an organic, inorganic or hybrid solid material, forming a repeating three-dimensional network capable of trapping one or more metal ions or complexes, modified by incorporation of at least one oxidizing species chosen from transition metals, in the form of salt, of oxide or of complexes with a ligand, to reduce the odour problems associated with processes for permanently reshaping the hair, the organic, inorganic or hybrid solid material being chosen from a zeolite or a zeotype.

## Patentansprüche

1. Nicht färbende, wässrige, reduzierende Zusammensetzung zur dauerhaften Verformung der Haare, umfassend, in einem kosmetisch verträglichen Medium:

   (i) mindestens ein Reduktionsmittel mit der Formel:

   $$(H)_m \cdot X(R)_n \qquad (I),$$

   worin X für P, S oder $SO_2$ steht, m' gleich 0 oder 1 ist, n gleich 1, 2 oder 3 ist und R ein linearer, verzweigter, gesättigter oder ungesättigter $(C_1-C_{20})$-Kohlenwasserstoffrest ist, der gegebenenfalls durch ein Heteroatom unterbrochen ist und gegebenenfalls Substituenten umfasst, ausgewählt aus einer Hydroxy-Gruppe, einer halogenierten Gruppe, einer Amin-Gruppe oder einer Carboxy-Gruppe, einer $((C_1-C_{30})$ Alcoxy) carbonyl-Gruppe, einer Amido-Gruppe, einer $((C_1-C_{30})$Alkyl)aminocarbonyl-Gruppe, einer $(C_1-C_{30})$Acyl)amino-Gruppe, einer Mo-no- oder Di $((C_1-C_{30})$alkyl) amino-Gruppe, einer Mono- oder Dihydroxy$((C_1-C_{30})$alkyl)amino-Gruppe, oder eines seiner Salze in Kombination mit einer Base, und
   (ii) mindestens einen Zeolithen oder Zeotypen, der ein dreidimensionales, sich wiederholendes Netzwerk bildet, das in der Lage ist, ein oder mehrere Ionen oder Metallkomplexe einzuschließen, modifiziert durch die Integration mindestens einer oxidierenden Spezies, ausgewählt aus den Übergangsmetallen, in Form von Salz, Oxid oder von Komplexen mit einem Liganden.

2. Zusammensetzung nach Anspruch 1, **dadurch gekennzeichnet, dass** der modifizierte Zeolith ein natürlicher oder künstlicher Zeolith ist, der der folgenden allgemeinen Formel entspricht:

   $$XM'_{2/n}O.Al_2O_3.YSiO_2.ZH_2O \qquad (II)$$

   worin M' ein austauschbares Ion ist, bevorzugt ein einwertiges oder zweiwertiges Metallion, n die Valenz des Ions M' ist, X ein Metalloxid ist, Y die Anzahl der Siliciumdioxidmoleküle ist und Z die Anzahl der Kristallisationswassermoleküle ist.

3. Zusammensetzung nach Anspruch 1 oder 2, **dadurch gekennzeichnet, dass** der modifizierte Zeolith ausgewählt ist aus einem Zeolithen des Typs A, K, L, P-L, O, T, X, Y und Ω, den Zeolithen mit hohem Siliciumdioxidanteil, Sodalith, Mordenit, Analzim, Crynoptyrolit, Chabasit und Erionit.

4. Zusammensetzung nach Anspruch 1 oder 2, **dadurch gekennzeichnet, dass** der modifizierte Zeolith ein synthetischer Zeolith ist.

5. Zusammensetzung nach einem der Ansprüche 1 bis 4, **dadurch gekennzeichnet, dass** M' für Na, K, Ca, Mg oder Ba steht.

**6.** Zusammensetzung nach einem der Ansprüche 1 bis 5, **dadurch gekennzeichnet, dass** der Zeolith modifiziert ist durch die Integration eines Metalls in Form eines Salzes, mit der Formel:

$$M(S)_n \qquad (II),$$

worin M ein Metall ist, ausgewählt aus Ti, V, Cr, Mn, Fe, Co, Ni, Cu, Zn, Mo, Ru, Rh, Pd und Ag; S ein anorganisches Anion ist, ausgewählt aus Sulfat-, Sulfonat-, Phosphat-, Phosphonat-, Chlorid-, Bromid- und Nitrationen; oder ein organisches Anion, ausgewählt aus Lactat-, Citrat-, Acetationen, und worin n gleich 1 bis 8 ist.

**7.** Zusammensetzung nach einem der Ansprüche 1 bis 5, **dadurch gekennzeichnet, dass** der Zeolith modifiziert ist durch die Integration eines Metalls in Form eines Oxids, mit der Formel:

$$M_{n'}(O)_n \qquad (III),$$

worin M ein Metall ist, ausgewählt aus Ti, V, Cr, Mn, Fe, Co, Ni, Cu, Zn, Mo, Ru, Rh, Pd und Ag; n' gleich 1 bis 5 ist, und O für Sauerstoff steht und n gleich 1 bis 8 ist.

**8.** Zusammensetzung nach einem der Ansprüche 1 bis 5, **dadurch gekennzeichnet, dass** der Zeolith modifiziert ist durch die Integration eines Metalls in Form eines Komplexes mit einem Liganden, wobei der Komplex der folgenden Formel entspricht:

$$M(L)_n \qquad (IV),$$

worin M ein Metall ist, ausgewählt aus Ti, V, Cr, Mn, Fe, Co, Ni, Cu, Zn, Mo, Ru, Rh, Pd und Ag; L ein organischer Ligand ist, ausgewählt aus gesättigten oder ungesättigten, cyclischen oder acyclischen Verbindungen, umfassend Carboxylatgruppen, primäre, sekundäre und tertiäre Amine, Hydroxy-Gruppen oder einen anorganischen Liganden, ausgewählt aus $NH_3$, NO, und worin n gleich 1 bis 10 ist.

**9.** Zusammensetzung nach Anspruch 8, **dadurch gekennzeichnet, dass** der organische Ligand ausgewählt ist aus: 1,4,8,11-Tetraazacyclotetradecan, 1,8-Diazabicyclo[5.4.0]undec-7-en, Ethylendiamin, Nitrilotriessigsäure, Tris(aminoethyl)amin, Tris(aminomethyl)methan, 1,3,5-Triaminocyclohexan, 2,6-Dicarboxylpyridin, Pyridin-2,6-dicarbonsäure, 2,2',6',2"-Terpyridin, 1,10-Phenanthrolin, Tris(2-pyridylmethyl)amin, Tris(2-pyridylethyl)amin und N-Carboxymethyl-N-(2-pyridylmethyl)glycin.

**10.** Zusammensetzung nach Anspruch 1, **dadurch gekennzeichnet, dass** der Feststoff ein Zeotyp ist, umfassend eine oxidierende Spezies, die integraler Bestandteil seines dreidimensionalen Netzwerks ist.

**11.** Zusammensetzung nach Anspruch 10, **dadurch gekennzeichnet, dass** die oxidierende Spezies ein Metall ist, ausgewählt aus Ti, V, Cr, Mn, Fe, Co, Ni, Cu, Zn, Mo, Ru, Rh, Pd und Ag.

**12.** Zusammensetzung nach Anspruch 11, **dadurch gekennzeichnet, dass** das Metall in Form eines Komplexes mit einem Liganden vorliegt, wobei der Komplex der folgenden Formel entspricht:

$$M(L)_n \qquad (IV),$$

worin M ein Metall ist, ausgewählt aus Ti, V, Cr, Mn, Fe, Co, Ni, Cu, Zn, Mo, Ru, Rh, Pd und Ag; L ein organischer Ligand ist, ausgewählt aus gesättigten oder ungesättigten, cyclischen oder acyclischen Verbindungen, umfassend Carboxylatgruppen, primäre, sekundäre und tertiäre Amine, Hydroxy-Gruppen oder einen anorganischen Liganden, ausgewählt aus $NH_3$, NO, und worin n gleich 1 bis 10 ist.

**13.** Zusammensetzung nach Anspruch 12, **dadurch gekennzeichnet, dass** der organische Ligand ausgewählt ist aus: 1,4,8,11-Tetraazacyclotetradecan, 1,8-Diazabicyclo[5.4.0]undec-7-en, Ethylendiamin, Nitrilotriessigsäure, Tris(aminoethyl)amin, Tris(aminomethyl)methan, 1,3,5-Triaminocyclohexan, 2,6-Dicarboxylpyridin, Pyridin-2,6-dicarbonsäure, 2,2',6',2"-Terpyridin, 1,10-Phenanthrolin, Tris(2-pyridylmethyl)amin, Tris(2-pyridylethyl)amin und N-Carboxymethyl-N-(2-pyridylmethyl)glycin.

**14.** Zusammensetzung nach einem der Ansprüche 1 bis 13, **dadurch gekennzeichnet, dass** der Feststoff oder die Feststoffe in einem Gehalt zwischen 0,1 und 10 Ges.-%, vorzugsweise zwischen 1 und 5 Ges.-%, bezogen auf das Gesamtgewicht der Zusammensetzung, vorliegt/vorliegen.

**15.** Zusammensetzung nach einem der Ansprüche 1 bis 14, **dadurch gekennzeichnet, dass** das oder die Reduktionsmittel ausgewählt sind aus Thioglycolsäure, Thiomilchsäure, Glycerinmonothioglycolat, Cysteamin, N-Acetylcysteamin, N-Propionylcysteamin, Cystein, N-Acetylcystein, Thioäpfelsäure, Panthetein, 2,3-Dimercaptobernsteinsäure, N-(Mercaptoalkyl)-ω-hydroxyalkylamiden, N-mono- oder N,N-Dialkylmercapto-4-butyramiden, Aminomercaptoalkylamiden, Derivaten von N-(Mercaptoalkyl)succinamidsäuren und N-(Mercaptoalkyl)succinimiden, Alkylaminomercaptoalkylamiden, dem azeotropen Gemisch von 2-Hydroxypropylthioglyconat und (2-Hydroxy-1-methyl) ethylthioglycolat, Mercaptoalkylaminoamiden, N-Mercaptoalkylalkandiamiden und Formamidinsulfinsäurederivaten und deren Salzen.

**16.** Zusammensetzung nach einem der Ansprüche 1 bis 15, **dadurch gekennzeichnet, dass** sie mindestens ein oberflächenaktives Mittel umfasst, ausgewählt aus Alkylsulfaten, Alkylbenzolsulfaten, Alkylethersulfaten, Alklysulfonaten, quaternären Ammoniumsalzen, Alkylbetainen, oxyethylenierten Alkylphenolen, Alkanolamiden von Fettsäuren, oxyethylenierten Fettsäureestern, Hydroxypropylethern.

**17.** Zusammensetzung nach Anspruch 16, **dadurch gekennzeichnet, dass** das oder die oberflächenaktive(n) Mittel höchstens 30 Gew.-%, vorzugsweise zwischen 0,5 und 10 Ges.-% des Gesamtgewichts der Zusammensetzung darstellen.

**18.** Zusammensetzung nach einem der Ansprüche 1 bis 17, **dadurch gekennzeichnet, dass** sie Wasser oder ein Gemisch aus Wasser und einem oder mehreren $C_1$-$C_6$-Niederalkoholen, vorzugsweise Ethanol, Isopropanol oder tert.-Butanol, umfasst.

**19.** Zusammensetzung nach einem der Ansprüche 1 bis 18, **dadurch gekennzeichnet, dass** sie eine wässrige Form, vorzugsweise die Form einer Lotion, verdickt oder nicht, einer Creme, verdickt oder nicht, oder eines Gels aufweist.

**20.** Verfahren zur dauerhaften Verformung der Haare, umfassend:

- einen Schritt zum Auftragen einer reduzierenden Zusammensetzung nach einem der Ansprüche 1 bis 18 auf die Haare, um die Disulfidbindungen des Keratins zu reduzieren,
- einen Schritt zum Fixieren durch Oxidation, um die Bindungen wieder herzustellen, durch Auftragen einer oxidierenden Zusammensetzung auf die Haare oder durch Inkontaktbringen der Haare mit Luftsauerstoff.

**21.** Verfahren nach Anspruch 20, **dadurch gekennzeichnet, dass** der modifizierte Stoff zum Zeitpunkt der Anwendung in die reduzierende Zusammensetzung integriert wird.

**22.** Verfahren zur dauerhaften Verformung der Haare, umfassend:

- einen Schritt zum Aufbringen einer wässrigen Zusammensetzung auf die Haare, umfassend, in einem kosmetisch verträglichen Medium, mindestens einen Zeolithen oder Zeotypen, der ein dreidimensionales, sich wiederholendes Netzwerk bildet, das in der Lage ist, ein oder mehrere Ionen oder Metallkomplexe einzuschließen, modifiziert durch die Integration mindestens einer oxidierenden Spezies, ausgewählt aus den Übergangsmetallen, in Form von Salz, Oxid oder von Komplexen mit einem Liganden,
- einen Schritt zum Aufbringen einer wässrigen reduzierenden Zusammensetzung auf die Haare, um die Disulfidbindungen des Keratins zu reduzieren, umfassend, in einem kosmetisch verträglichen Medium, mindestens ein Reduktionsmittel mit der Formel:

$$(H)_m \cdot X(R)_n \qquad (I),$$

worin X für P, S oder $SO_2$ steht, m' gleich 0 oder 1 ist, n gleich 1, 2 oder 3 ist und R ein linearer, verzweigter, gesättigter oder ungesättigter $(C_1$-$C_{20})$-Kohlenwasserstoffrest ist, der gegebenenfalls durch ein Heteroatom unterbrochen ist und gegebenenfalls Substituenten umfasst, ausgewählt aus einer Hydroxy-Gruppe, einer halogenierten Gruppe, einer Amin-Gruppe oder einer Carboxy-Gruppe, einer $((C_1$-$C_{30})$Alcoxy) carbonyl-Gruppe, einer Amido-Gruppe, einer $((C_1$-$C_{30})$Alkyl)aminocarbonyl-Gruppe, einer $(C_1$-$C_{30})$Acyl)amino-Gruppe, einer Mono- oder Di $((C_1$-$C_{30})$ alkyl) amino-Gruppe, einer Mono- oder Dihydroxy$((C_1$-$C_{30})$ alkyl) amino-Gruppe, oder eines seiner Salze in Kombination mit einer Base, und
- der Zeolith oder Zeotyp vor dem Auftragen der reduzierenden Zusammensetzung auf die Haare aufgebracht wird,
- einen Schritt zum Fixieren durch Oxidation, um die Disulfidbindungen wieder herzustellen, durch Auftragen einer oxidierenden Zusammensetzung auf die Haare oder durch Inkontaktbringen der Haare mit Luftsauerstoff.

23. Verfahren nach einem der Ansprüche 20 bis 22, **dadurch gekennzeichnet, dass** man die reduzierende Zusammensetzung 5 bis 60 Minuten lang, vorzugsweise 15 bis 45 Minuten lang einwirken lässt.

24. Verfahren nach einem der Ansprüche 20 bis 23, **dadurch gekennzeichnet, dass** die oxidierende Zusammensetzung mindestens ein Oxidationsmittel umfasst, das aus Wasserstoffperoxid, Alkalibromaten, Persalzen, Polythionaten und einem Gemisch aus Alkalibromat und Persalz ausgewählt ist.

25. Verfahren nach einem der Ansprüche 20 bis 24, **dadurch gekennzeichnet, dass** es zwischen dem Schritt zum Auftragen der reduzierenden Zusammensetzung auf die Haare und dem Schritt zum Fixieren einen Schritt zum Erwärmen der Haare mit einem Frisierstab auf eine Temperatur zwischen 60 und 250 °C, vorzugsweise zwischen 120 und 220 °C umfasst.

26. Kit zur dauerhaften Verformung der Haare, umfassend in einem ersten Kompartiment eine reduzierende Zusammensetzung nach einem der Ansprüche 1 bis 19 und in einem zweiten Kompartiment eine oxidierende Zusammensetzung.

27. Verwendung einer reduzierenden Zusammensetzung nach einem der Ansprüche 1 bis 19 aus einem organischen, anorganischen oder hybriden Feststoff, der ein dreidimensionales, sich wiederholendes Netzwerk bildet, das in der Lage ist, ein oder mehrere Ionen oder Metallkomplexe einzuschließen, modifiziert durch die Integration mindestens einer oxidierenden Spezies, ausgewählt aus Übergangsmetallen, in Form von Salz, Oxid oder von Komplexen mit einem Liganden, um die Geruchsprobleme zu reduzieren, die mit den Verfahren zur dauerhaften Verformung der Haare verbunden sind, wobei der organische, anorganische oder hybride Feststoff ausgewählt ist aus einem Zeolithen oder einem Zeotypen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5184630 A
- EP 354835 A
- EP 368763 A
- EP 432000 A
- EP 465342 A
- EP 514282 A
- FR 2679448 A
- FR 2692481 A
- EP 653202 A
- FR 2598613
- FR 2470596

- FR 2535730
- US 4749732 A
- GB 2197352 A
- FR 1530369
- EP 295780 A
- FR 7932078
- FR 2472382
- WO 8026421 A
- FR 2495931
- US 4480135 A
- US 5110995 A